# EUROPÄISCHE PATENTSCHRIFT

(11) **EP 1 200 825 B1**
(45) Veröffentlichungstag und Bekanntmachung des Hinweises auf die Patenterteilung: **24.08.2005**
(21) Anmeldenummer: 00956283.6
(22) Anmeldetag: 25.07.2000
(51) Int. Cl.: G01N 33/53

(54) **RELAXATION DER DOPPELBRECHUNG MAGNETISCHER NANOPARTIKEL WÄHREND BINDUNGSREAKTIONEN**
METHOD FOR DETECTING BINDING REACTIONS THROUGH MEASUREMENT OF THE RELAXATION OF BIREFRINGENCE OF MAGNETIC NANOPARTICLES
PROCEDE DE DETECTION DE REACTIONS DE LIAISON A L'AIDE DE LA MESURE DE LA RELAXATION DE LA BIREFRINGENCE DE PARTICULES MAGNETIQUES

(30) Priorität: 06.08.1999 DE 19938384
(43) Veröffentlichungstag der Anmeldung: 02.05.2002
(73) Patentinhaber: Institut für Diagnostikforschung GmbH an der Freien Universität Berlin, 13353 Berlin (DE)
(72) Erfinder: KÖTITZ, Roman, D-07749 Jena (DE); LANGE, Julia, D-12163 Berlin (DE); BROWAEYS, Julien, F-75018 Paris (FR); PERZYNSKI, Régine, F-75010 Paris (FR); BACRI, Jean-Claude, F-75015 Paris (FR); PONSINET, Virginie, F-94370 Sucy-en-Brie (FR); RHEINLÄNDER, Thomas, D-79541 Lörrach (DE)
(74) Vertreter: Seuss, Thomas
(86) Internationale Anmeldenummer: PCT/EP2000/007150
(87) Internationale Veröffentlichungsnummer: WO 2001/011360

(56) Entgegenhaltungen:
- DE-A- 19 503 664
- KOTITZ R ET AL: "Determination of the binding reaction between avidin and biotin by relaxation measurements of magnetic nanoparticles" JOURNAL OF MAGNETISM AND MAGNETIC MATERIALS,NL,ELSEVIER SCIENCE PUBLISHERS, AMSTERDAM, Bd. 194, Nr. 1-3, April 1999 (1999-04), Seiten 62-68, XP004166647 ISSN: 0304-8853
- WEITSCHIES W ET AL: "Determination of relaxing or remanent nanoparticle magnetization provides a novel binding-specific technique for the evaluation of immunoassays." PHARMACEUTICAL AND PHARMACOLOGICAL LETTERS, Bd. 7, Nr. 1, 1997, Seiten 5-8, XP002112220 ISSN: 0939-9488
- BACRI J -C ET AL: "Magnetic transient birefringence of ferrofluids: particle size determination" JOURNAL DE PHYSIQUE, AUG. 1987, FRANCE, Bd. 48, Nr. 8, Seiten 1385-1391, XP000980678 ISSN: 0302-0738 in der Anmeldung erwähnt
- BACRI J -C ET AL: "Ferrofluid viscometer" JOURNAL DE PHYSIQUE LETTRES, 15 DEC. 1985, FRANCE, Bd. 46, Nr. 24, Seiten L1199-L1205, XP002158068 ISSN: 0302-072X in der Anmeldung erwähnt
- HASMONAY E ET AL: "Optical properties of nickel ferrite ferrofluids" JOURNAL OF MAGNETISM AND MAGNETIC MATERIALS,NL,ELSEVIER SCIENCE PUBLISHERS, AMSTERDAM, Bd. 201, Nr. 1-3, Juli 1999 (1999-07), Seiten 195-199, XP004181232 ISSN: 0304-8853

## Beschreibung

Die Erfindung betrifft ein neues in-vitro-Verfahren zur Detektion von Analyten bzw. Bindungsreaktionen, bei denen ferro- oder ferrimagnetische Substanzen als Markierung in Immunoassays oder sonstigen Bindungsassays eingesetzt werden, welches dadurch gekennzeichnet ist, daß die Relaxation der Doppelbrechung als Meßgröße bestimmt wird, sowie die Verwendung der geeigneten ferro- oder ferrimagnetischen Substanzen in diesem Verfahren.

In der deutschen Patentschrift DE 195 03 664 C2 wird ein Verfahren zur magnetorelaxometrischen quantitativen Detektion von Analyten in Flüssig- und Festphasen beschrieben. Es handelt sich dabei um ein Assayverfahren, bei dem zunächst strukturspezifische Substanzen mit frei beweglichen ferri- oder ferromagnetischen kolloidalen Teilchen geeigneter magnetischer Relaxationszeit und mit geeignetem magnetischem Moment markiert werden und anschließend diese markierten strukturspezifischen Substanzen in einer zu vermessenden flüssigen oder immobilisierten Probe eingesetzt werden. Die zu vermessende Probe wird dann mittels eines von außen angelegten Magnetfeldes aufmagnetisiert, und nach Abschalten des äußeren Feldes wird die Relaxation der Magnetisierung der kolloidalen Teilchen mittels geeigneter Magnetfeldsensoren vermessen, wobei die durch spezifische Bindung veränderte Relaxationszeit und/oder die durch das Ausmaß der Bindung veränderte Relaxationsamplitude zur Analyse herangezogen wird. Auf diese Weise ist es z.B. möglich, quantitativ die Konzentration eines Antikörpers gegen Collagen zu bestimmen.

Der Nachteil der Methode besteht unter anderem darin, daß der Versuchsaufbau zur Durchführung des Verfahrens sehr aufwendig ist. Zur Messung des Magnetfeldes werden SQUIDs (Superconducting QUantum Interference Devices) verwendet, die sich in einem speziellen Kryostaten befinden und aufwendig mit flüssigem Helium gekühlt werden müssen. Hinzu kommt, daß für hochempfindliche Messungen magnetische Störfelder unterdrückt werden müssen, weshalb das Verfahren derzeit nur in einem sehr aufwendig magnetisch abgeschirmten Raum durchgeführt werden kann. Weitere Probleme der der magnetischen Meßtechnik ergeben sich durch eine technisch bedingte Totzeit, wodurch Informationen über den entscheidenden Anfangsteil des Relaxationssignals nicht zugänglich sind, sowie ein Untergrundsignal, das kompensiert werden muß.

Weiter ist bekannt, daß Ferrofluide Doppelbrechung zeigen, wenn sich bei Anlegen eines magnetisierenden Feldes die ferri- oder ferromagnetischen kolloidalen Teilchen des Ferrofluids als Ganzes in Richtung des Feldes ausrichten. Nach Abschalten des Feldes kommt es dann durch thermische Reorientierung der magnetischen Teilchen zu einer Relaxation der Doppelbrechung. Dieses Relaxationssignal ist auch als "magnetic transient birefringence" bekannt. Die Zeitkonstante dieses Relaxationsprozesses hängt von der Temperatur, der Viskosität der Trägerflüssigkeit und dem hydrodynamischen Volumen der magnetischen Teilchen ab. Durch Messung der Relaxation der Doppelbrechung kann dann das hydrodynamische Volumen der magnetischen Teilchen bestimmt werden (siehe Bacri et al., "Magnetic transient birefringence of ferrofluids: particle size determination", J. Physique 48 (1987), 1385-1391). Sowohl in Bacri et al als auch in Hasmonay et al (Journal of Magnetism and Magnetic Materials, Bd. 201, Nr. 1-3, Juli 1999, Seiten 195-199) werden Messungen an kolloidalen Lösungen beschrieben, bei denen die ferromagnetischen Teilchen in einen Volumenanteil von < 2% bzw. mindestens 0,23% in der Lösung vorliegen.

Aufgabe der vorliegenden Erfindung ist es, ein neues in-vitro-Verfahren zur Detektion von Analyten bzw. Bindungsreaktionen unter Verwendung magnetischer Teilchen zu entwickeln, das die Nachteile des bekannten Verfahrens nach DE 195 03 664 C2 überwindet, insbesondere wesentlich einfacher durchzuführen ist, und eine Detektion der Analyten bzw. Bindungsreaktionen mit einer vergleichbaren Empfindlichkeit ermöglicht.

Diese Aufgabe wird durch die vorliegende Erfindung gelöst, welche in den Patentansprüchen definiert wird.

Es wurde gefunden, daß die Detektion von Analyten bzw. Bindungsreaktionen in Flüssigphasen gelingt, wenn ferro- oder ferrimagnetische Substanzen als Markierung in Immunoassays oder sonstigen Bindungsassays eingesetzt werden und die Relaxation der Doppelbrechung als Meßgröße bestimmt wird. Das neue Verfahren detektiert nicht mehr das durch die Reorientierung der magnetischen Momente der ferro- oder ferrimagnetischen Substanzen abklingende magnetische Feld der Probe, sondern die Relaxation der Doppelbrechung als Maß für die Reorientierung der ferro- oder ferrimagnetischen Substanzen in der Probe. Es wurde überraschend gefunden, daß die Empfindlichkeit der Messung der Relaxation der Doppelbrechung mit der Empfindlichkeit der direkten Messung der Relaxation der Magnetisierung der Probe vergleichbar ist.

Das neue Verfahren beruht auf einer speziellen Meßtechnik, die es erlaubt, nach Ausrichtung der ferro- oder ferrimagnetischen Substanzen durch Anlegen eines äußeren Magnetfeldes in der Probe eine Doppelbrechung zu erzeugen und nach Abschalten des äußeren Magnetfeldes die Relaxation der Doppelbrechung zu bestimmen. Die oben geschilderten Nachteile der bekannten Meßtechnik werden mit dem neuen Verfahren überwunden.

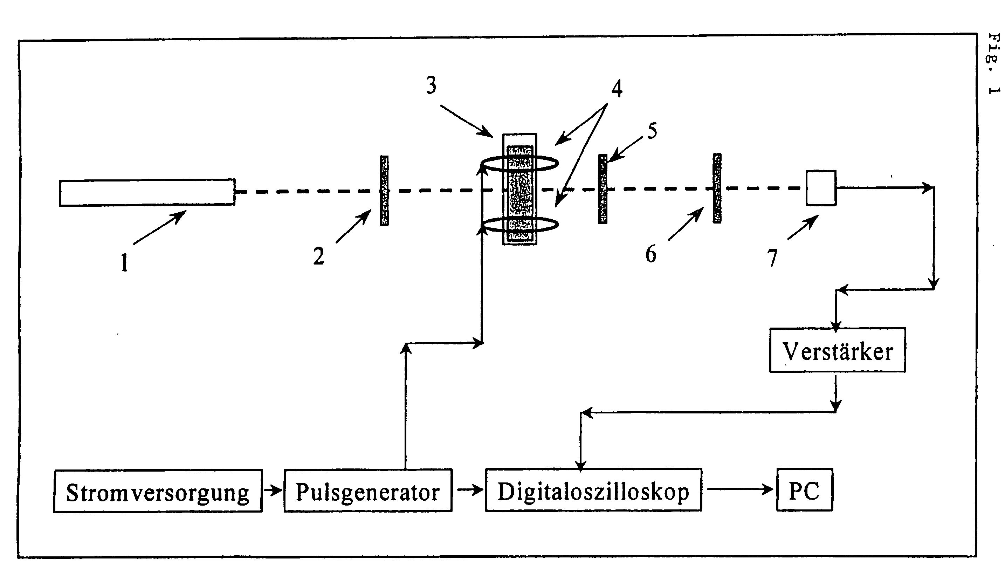

Das Verfahren wird mit einer Meßanordnung durchgeführt, die zunächst eine Ausrichtung der ferro- oder ferrimagnetischen Substanzen der zu untersuchenden Probe mittels eines geeigneten Magnetfeldes erlaubt und anschließend die Messung der Relaxation der Doppelbrechung dieser Substanzen ermöglicht.
Diese Meßanordung enthält im allgemeinen eine Vorrichtung zur Erzeugung polarisierten Lichtes, eine Vorrichtung zur Aufnahme der Probe, eine Vorrichtung zur Aufmagnetisierung der Probe mit Magnetpulsen oder einem Magnetfeld variabler Frequenz, sowie eine Vorrichtung zur Analyse der Polarisationsrichtung polarisierten Lichtes.
Ein Ausführungsbeispiel für eine Vorrichtung, die zur Durchführung des Verfahrens geeignet ist, umfaßt z. B. eine optische Bank, auf der ein Laser, ein Polarisator, eine Küvette mit der Probe, ein Analysator und ein Detektor angeordnet sind. Die Probe wird in eine Magnetisierungsspule eingebracht, die über eine Stromversorgung und einen Pulsgenerator angesteuert wird, und das Meßsignal wird zur Auswertung zu einer Datenverarbeitungsanlage geführt. Eine solche Vorrichtung ist schematisch in Fig. 1 dargestellt. Dabei zeigt (1) einen He-Ne-Laser, (2) einen Polarisator, (3) eine Küvette mit Probe, (4) die Magnetisierungsspulen, (5) eine λ/4-Platte (optional), (6) einen Analysator und (7) einen Photodetektor. Eine derartige Vorrichtung wird z.B. in J. Physique Lett., Vol. 46, 1985, L-1199 - L-1205, beschrieben.

Besonders empfindlich kann nach Aufmagnetisierung der Probe (Stärke der Pulse z.B. 10 mT, Dauer z.B. 2 ms, Pause z.B. 20 ms) und nach Abschalten des Feldes die Relaxation der Doppelbrechung mittels hochempfindlicher Photosensoren, wie z.B. Pin-Dioden oder Avalanche-Dioden, gemessen werden. Zur Verbesserung des Signal-Rausch-Verhältnisses werden mehrere Messungen gemittelt.
Alternativ zur Messung der Relaxation der Doppelbrechung im Zeitbereich kann auch eine Messung der Doppelbrechung im Frequenzbereich durchgeführt werden. Dabei wird eine Reihe von Messungen vorgenommen, bei denen die Probe magnetischen Wechselfeldern ausgesetzt ist und die Doppelbrechung in Bezug auf das magnetisierende Feld mit Betrag und Phase detektiert wird. Diese Meßgröße kann in eine komplexe Form transformiert werden, wobei aus Real- und Imaginärteil dieser komplexen Doppelbrechung Informationen über die Relaxationszeiten der Doppelbrechung gewonnen werden können.

Wie bereits in der deutschen Patentschrift DE 195 03 664 C2 beschrieben wurde, relaxiert die Magnetisierung frei beweglicher ferro- oder ferrimagnetischer kolloidaler Teilchen nach Abschalten eines äußeren magnetisierenden Feldes innerhalb der Meßzeit durch zwei verschiedene Mechanismen:
(i) Drehung des gesamten kolloidalen Teilchens innerhalb der umgebenden Flüssigkeit, wobei die Zeitkonstante vom hydrodynamischen Durchmesser der Teilchen, der Viskosität der Trägerflüssigkeit und der Temperatur abhängt, was hauptsächlich Parameter der Umgebung der Teilchen reflektiert, dieser Mechanismus wird im folgenden auch als Brownsche Relaxation bezeichnet
   und
(ii) Drehung des internen Magnetisierungsvektors innerhalb des magnetischen Kerns der kolloidalen Teilchen, wobei die Zeitkonstante sehr empfindlich von Material und Form (Anisotropiekonstante des verwendeten Teilchenmateriais), Volumen und der Temperatur des magnetischen Kerns der verwendeten Teilchen abhängt. Dies sind im wesentlichen intrinsische Parameter der Partikel, dieser Mechanismus wird auch als Néelsche Relaxation bezeichnet.

Diese beiden Mechanismen bestimmen ebenfalls die Veränderung der Magnetisierung eines Systems frei beweglicher ferro- oder ferrimagnetischer kolloidaler Teilchen bei Einschalten eines äußeren Magnetfeldes.

In Abwesenheit eines äußeren Magnetfeldes zeigen Ferrofluide keine Doppelbrechung. Voraussetzung für die Erzeugung der Doppelbrechung ist die Drehung des gesamten Teilchens durch ein äußeres Magnetfeld, mit anderen Worten eine Reaktion auf das angelegte Magnetfeld nach dem Brownschen Mechanismus. Teilchen, die nach dem Néel-Mechanismus auf ein äußeres Magnetfeld reagieren, tragen nicht zur Doppelbrechung bei. Dominierend ist der Prozeß mit der kürzeren Relaxationszeit. Deshalb tragen zur Doppelbrechung nur Teilchen bei, deren Brown-Relaxationszeit kürzer ist als ihre Neel-Relaxationszeit.

Wenn in Immunoassays oder sonstigen Bindungsassays ferro- oder ferrimagnetische Substanzen eingesetzt werden, deren Brownsche Relaxation unter den Meßbedingungen im ungebundenen Zustand schneller verläuft als die Néelsche Relaxation, kann durch die Änderung des dominierenden Relaxationsmechanismus beziehungsweise durch die Vergrößerung des Teilchenvolumens, welche durch die Bindung eintritt, der Anteil gebundener magnetischer Marker neben gleichzeitig in der Meßprobe vorhandenen ungebundenen magnetischen Markern bestimmt werden.

Wie bei dem in DE 195 03 664 C2 beschriebenen Verfahren werden die die Analyten bindenden strukturspezifischen Substanzen zunächst mit ferrimagnetischen oder ferromagnetischen kolloidalen Teilchen markiert. Diese magnetisch markierten strukturspezifischen Substanzen werden der zu vermessenden flüssigen oder immobilisierten Probe beigesetzt und die zu vermessende Probe mittels eines von außen angelegten Magnetfeldes aufmagnetisiert. Nach Abschalten des äußeren Feldes wird die Relaxation der Doppelbrechung oder die Doppelbrechung im Frequenzbereich bestimmt.

Das Verfahren wird bevorzugt so durchgeführt, daß
(i) die die Analyten bindenden strukturspezifischen Substanzen zunächst mit ferrimagnetischen oder ferromagnetischen Substanzen markiert werden und anschließend
(ii) diese markierten strukturspezi fischen Substanzen in einer zu vermessenden Probe eingesetzt werden,
(iii) die zu vermessende Probe mittels eines von außen angelegten Magnetfeldes aufmagnetisiert wird und
(iv) nach Abschalten des äußeren Feldes die Relaxation der Doppelbrechung der magnetischen Marker vermessen wird.

Das Verfahren kann auch so durchgeführt werden, daß
(i) Analyte zunächst mit ferrimagnetischen oder ferromagnetischen Substanzen markiert werden und anschließend
(ii) diese magnetisch markierten Analyte in einer zu vermessenden Probe eingesetzt werden, welcher Substanzen zugesetzt wurden, die die Analyte spezifisch binden, und
(iii) die zu vermessende Probe mittels eines von außen angelegten Magnetfeldes aufmagnetisiert wird und
(iv) nach Abschalten des äußeren Feldes die Relaxation der Doppelbrechung der magnetischen Marker vermessen wird.

Die Auswertung der Messergebnisse erfolgt hier, wie auch bei den nachfolgend beschriebenen kompetitiven Assayverfahren, in einer dem Fachmann bekannten Weise, d.h. analog zu den Verfahren wie sie bei Immunoassays oder Radioassays angewandt werden.

In beiden vorgenannten Fällen kann zur Analyse auch die Messung der durch die Bindung veränderten Doppelbrechung im Frequenzbereich herangezogen werden.

Die bisher nur in Ausnahmefällen mögliche Diskriminierung zwischen gebundenen und ungebundenen Markern wird durch die Ausnutzung ihrer unterschiedlichen Relaxationsmechanismen oder die durch die Bindung verursachte Beeinflussung der Relaxationszeit des magnetischen Markers ermöglicht.

In flüssiger Phase können Analyte dadurch nachgewiesen werden, daß die die Analyten bindenden strukturspezifischen Substanzen zunächst
(i) mit ferrimagnetischen oder ferromagnetischen Substanzen markiert werden, wobei diese Substanzen so gewählt werden, daß die Brownsche Relaxation zumindest eines Teils dieser Substanzen unter den Meßbedingungen eine kürzere Relaxationszeit aufweist als die Néelsche Relaxation und anschließend
(ii) diese magnetisch markierten Substanzen in einer zu vermessenden Probe eingesetzt werden, und
(iii) die zu vermessende Probe mittels eines von außen angelegten Magnetfeldes geeigneter Stärke aufmagnetisiert wird und
(iv) nach Abschalten des äußeren Feldes die Relaxation der Doppelbrechung vermessen wird, wobei das unterschiedliche Relaxationsverhalten der mit dem Analyten gebundenen gegenüber den ungebundenen magnetischen Markern zur Analyse herangezogen wird.

Als Meßgröße kann auch die Doppelbrechung der Probe im Frequenzbereich bestimmt werden.

Auch in diesem Fall ist es möglich, anstelle strukturspezifischer Substanzen die nachzuweisenden Analyte mit den magnetischen Markierungen zu kombinieren.

Unter strukturspezifischen Substanzen sind alle Substanzen zu verstehen, die spezifisch an bestimmte Strukturen binden. Unter strukturspezifischen Substanzen sind insbesondere Antikörper, Antikörperfragmente, Biotin oder Biotin bindende Substanzen wie Avidin bzw. Streptavidin, Extravidin ode Neutravidin, spezifisch an Rezeptoren bindende Agonisten wie Zytokine, Lymphokine, Endotheline oder deren Antagonisten, spezifische Peptide und Proteine, Rezeptoren, Enzyme, Enzymsubstrate, Nukleotide, Ribonukleinsäuren, Desoxyribonukleinsäuren, Kohlenhydrate, Lipoproteine etc. zu verstehen. Als strukturspezifische Substanzen sind Substanzen bevorzugt, deren Bindungskonstante im Bereich von 10⁵ - 10¹⁵ (mol/l)⁻¹ liegt. Insbesonders bevorzugt sind Substanzen deren Bindungskonstante im Bereich von 10⁷ - 10¹⁵ (mol/l)⁻¹ liegt.

Die strukturspezifischen Substanzen oder nachzuweisenden Analyte lassen sich mit Hilfe von in der Immuno-, Peptid- und Proteinchemie geläufigen Verfahren mit den ferri- oder ferromagnetischen Teilchen markieren. Besonders vorteilhaft sind kovalente Bindungen zwischen den strukturspezifischen Substanzen beziehungsweise den nachzuweisenden Analyten mit den die stabilisierende Hülle der ferri- oder ferromagnetischen Teilchen bildenden Substanzen. Beispiele für besonders geeignete Methoden sind die Aktivierung und Kopplung mittels Carbodiimiden [Jakoby and Wilchek, eds.; Methods Enzymol. (1974) 34], die Bildung von Schiffschen Basen nach Einwirkung von Periodaten auf Kohlenhydrate enthaltende Verbindungen (Wichek and Bayer, eds., Methods Enzym 184:177), die gegebenenfalls zur weiteren Stabilisierung anschließend noch reduziert werden, die Kopplung mittels Glutardialdehyd [Heitzmann and Richards, Proc. Natl. Acad. Sci. USA 71 (1974) 3537], die Quervemetzung bromoacetylierter Teilchen mit thiolylierten Substanzen [Angerer et al.; Cell 9 (1976) 81], sowie die reduktive Alkylierung (Bayer et al.; J. Histochem. Cytochem. 24 (1976) 933].

Als Substanzen für die magnetische Markierung können alle ferromagnetischen oder ferrimagnetischen Materialien verwendet werden, die sich in einem zur Detektion geeigneten Medium dispergieren lassen, wobei die Néelsche Relaxationszeit zumindest eines Teils der magnetischen Markierungen unter den Meßbedingungen größer als die Brownsche Relaxationszeit dieser magnetischen Markierungen ist. Besonders geeignet sind alle ferromagnetischen oder ferrimagnetischen kolloidalen Teilchen mit Brownschen Relaxationszeiten in wäßrigen Medien im Bereich von 10⁻⁸ - 10⁻¹ s und entsprechend längeren Néelschen Relaxationszeiten. Für die Durchführung der Messungen muß die Viskosität des verwendeten Dispergiermediums mit den Relaxationszeiten der ferromagnetischen und ferrimagnetischen Teilchen und der Meßzeit abgestimmt werden, da das Suspensionsmedium wesentlich die Zeitkonstante der Brownschen Relaxation bestimmt. Dabei berücksichtigt man die Temperaturabhängigkeit der Viskosität des Dispergiermediums.

Bevorzugt sind insbesondere ferromagnetische oder ferrimagnetische kolloidale Teilchen aus Eisen, Eisenoxiden, Bariumferriten, Strontiumferriten, Kobalt, Nickel, Nickelferriten, Kobaltferriten und Chromdioxid, deren Néelsche Relaxationszeit größer als die Brownsche Relaxationszeit ist.

Die Verwendung von magnetischen Markierungen mit eng verteilten Partikelgrößen und/oder magnetischen Momenten bzw. Brownschen und Néelschen Relaxationszeiten ist im allgemeinen vorteilhaft. Eine Auftrennung magnetischer Markierungen in Fraktionen mit enger Verteilung der Partikelgrößen kann z.B. durch chromatographische Verfahren oder unter Verwendung spezieller Filtrationsverfahren (z.B. Glaskapillarsysteme oder Tangentialfiltration), durch die Verwendung von Molekularsieben oder mittels Zentrifugation erreicht werden. Magnetische Markierungen mit möglichst einheitlichen Momenten lassen sich z.B. durch Klassierung in einem magnetischen Gradientenfeld herstellen.

Die ferromagnetischen und ferrimagnetischen Substanzen können mit einer Hülle aus oligomeren oder polymeren Kohlenhydraten, Proteinen, Peptiden, Nukleotiden, Tensiden, sonstigen Monomeren, Oligomeren oder Polymeren und/ oder Lipiden stabilisiert sein.

Die Teilchengrößen der ferromagnetischen und ferrimagnetischen Substanzen liegen vorteilhafterweise zwischen 1 nm und 100 µm. Bevorzugt sind kolloidale Teilchen mit Teilchengrößen zwischen 1 nm und 400 nm. Besonders bevorzugt sind Teilchengrößen zwischen 1 nm und 100 nm.

Als magnetische Marker können auch ferromagnetische oder ferrimagnetische Substanzen mit einer stabilisierenden Hülle aus der strukturspezifischen Substanz oder dem nachzuweisenden Analyten hergestellt werden, indem die Teilchen nach Herstellung direkt in eine Lösung der strukturspezifischen Substanz, gegebenenfalls in Gegenwart weiterer Hilfsstoffe wie z.B. Proteine, Kohlenhydrate sowie natürliche, synthetische oder partialsynthetische oberflächenaktive Substanzen usw. gebracht werden, bzw. direkt in Gegenwart der strukturspezifischen Substanzen hergestellt werden.

Geeignete magnetische Marker und diese Teilchen enthaltende Suspensionen werden beispielsweise in der WO 92/12735, der WO 92/22586, der EP 0 186 616 und der US 4,101,435 beschrieben. Es können prinzipiell auch magnetische Teilchen verwendet werden, die üblicherweise als Kontrastmittel für die Kernresonanztomographie verwendet werden, wie z.B. Resovist, Lumirem, Feridex, Combidex, Abdoscan und Clariscan.

Verbindungen, die aus kolloidalen Suspensionen frei beweglicher ferrimagnetischer oder ferromagnetischer Teilchen und strukturspezifischen Substanzen beziehungsweise nachzuweisender Analyte bestehen, wurden bereits in der deutschen Patentschrift DE 195 03 664 C2 beschrieben.

Die Verbindungen können auch aus Kombinationen mehrerer ferromagnetischer oder ferrimagnetischer Teilchen mit diskriminierbaren Relaxationszeiten bestehen, da bei Verwendung von unterschiedlichen magnetischen Markierungen mit jeweils sehr enger Verteilung der Relaxationszeiten und/oder magnetischen Momenten für verschiedene strukturspezifische Substanzen, bzw. Analyte innerhalb einer Probe individuell diskriminierbare Meßergebnisse erzielt werden können. Dadurch wird eine direkte simultane Bestimmung mehrerer Analyte ermöglicht.

Als Suspensionsmedien kommen alle Flüssigkeiten in Betracht, in denen die Verbindungen frei beweglich sind. Besonders geeignet sind Wasser, wäßrige Lösungen grenzflächenaktiver Hilfsstoffe, wie z.B. Tenside oder oligomere oder polymere Kohlenhydrate und Proteine, sowie Mischungen aus Wasser mit Alkoholen wie z.B. Glycerol und Polyethylenglykol. Die Suspensionsmedien können zusätzlich den osmotischen Druck verändernde Hilfsstoffe wie z.B. Kochsalz enthalten. Desweiteren können den pH-Wert bestimmende Puffersubstanzen, wie z.B. Phosphate, enthalten sein. Besonders bevorzugt sind Suspensionsmedien mit geeigneten optischen Eigenschaften wie geringer Absorption und Doppelbrechung. Alternativ kann die Lichtquelle so gewählt werden, daß das Licht möglichst wenig vom Suspensionsmedium absorbiert wird und insbesondere auch biologische Suspensionsmedien verwendet werden können.

Die Verbindungen aus den ferromagnetischen oder ferrimagnetischen kolloidalen Teilchen mit strukturspezifischen Substanzen oder nachzuweisenden Analyten können auch in getrockneter Form, gegebenenfalls in Kombination mit weiteren Hilfsstoffen die z.B. die Trocknung erleichtern oder die Stabilität des getrockneten Produkts erhöhen, vorliegen (z. B. als Lyophilisate) und erst kurz vor der Messung in das Suspensionsmedium überführt werden.

Aufgrund des auf physikalischen Mechanismen beruhenden Bindungsnachweises können unspezifische Meßsignale (Matrixphänomene) weitgehend ausgeschlossen werden. Die Spezifität des Verfahrens hängt somit nur noch von der "wahren" Spezifität der strukturspezifischen Substanz (Kreuzreaktivität von Antikörpern, unspezifische Bindung von Liganden) ab. Aufgrund der hohen Sensitivität des erfindungsgemäßen Verfahrens werden die sonst üblichen Detektionsgrenzen von Bindungsassays mühelos unterschritten.

Das erfindungsgemäße Verfahren kann z.B. in der Fertilität, Histokompatibilität, Allergologie, Infektiologie, Hygiene, Genetik, Virologie, Bakteriologie, Toxikologie, Pathologie, Umweltanalytik, Lebensmittelchemie und medizinischen Diagnostik zum Einsatz kommen.

Die nachfolgenden Beispiele dienen der näheren Erläuterung des Erfindungsgegenstandes, ohne ihn auf diese beschränken zu wollen.

### Beispiele

### 1. Messung einer Verdünnungsreihe von Dextran-Magnetit:

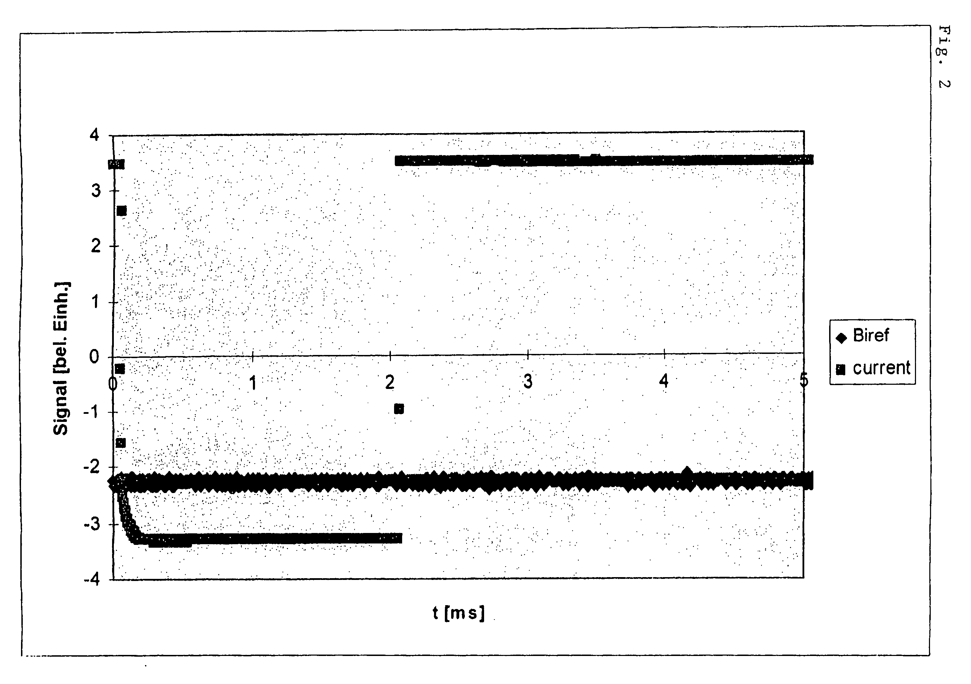

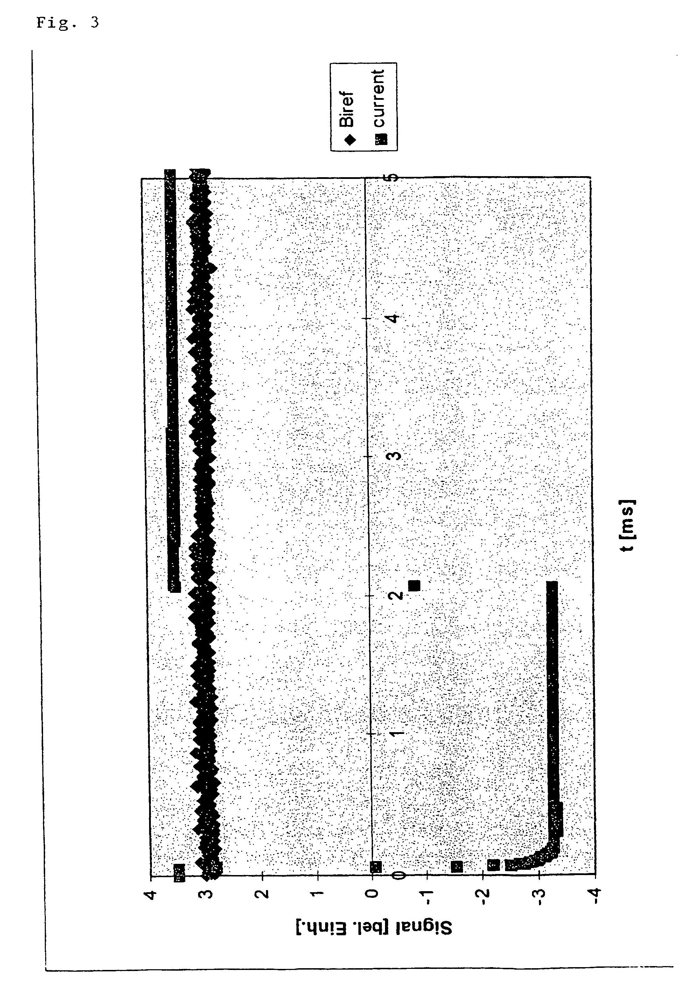

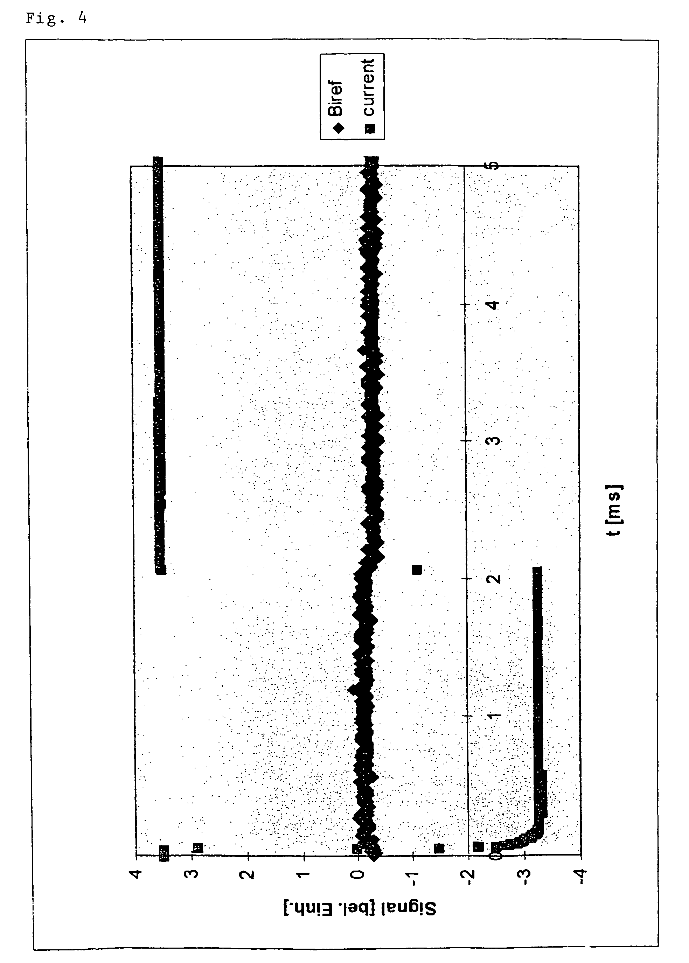

*Vorgehen:* Die Ausgangsprobe (mit Dextran gecoatetes Eisenoxid, hergestellt nach der Methode von M. Hasegawa and S. Hakukoku, U.S. Patent 4101435 (1978), Hersteller: Meito Sangyo) lag in einer Konzentration von 1 mol Fe/l vor. Daraus wurden jeweils um den Faktor 10 mit dest. Wasser verdünnte Proben hergestellt (Konzentrationsbereich 10⁻¹ - 10⁻⁶ mol Fe/l). Es wurde ebenfalls eine Vergleichsprobe mit destilliertem Wasser untersucht. Zur Messung wurde von allen Proben ein Volumen von 1 ml in die Küvette gefüllt.
Alle hier dargestellten Messungen wurden mit dem gleichen Protokoll durchgeführt: Die Proben wurden für eine Dauer von 2 ms wiederholt mit einem Magnetpuls der Stärke 100 Oe (10 mT) aufmagnetisiert. Die Pause zwischen den Pulsen betrug 20 ms. Zur Verbesserung des Signal-Rauschverhältnisses wurden 256 Einzelmessungen gemittelt.
*Ergebnis:* Für die Vergleichsprobe mit destilliertem Wasser und die Probe der Konzentration 10⁻⁶ mol Fe konnte kein Relaxationssignal detektiert werden (Vgl. Fig. 2, Relaxation in aqua dest, bzw. Fig. 3, Relaxation der Probe mit 10⁻⁶ mol Fe/l).
Die Relaxation einer Probe der Konzentration von 10⁻⁵ mol/ l Fe war detektierbar und ist in Fig. 4 gezeigt.

Die im Probenvolumen von 1 ml enthaltene Stoffmenge Fe dieser Probe beträgt 10 nmol. Beachtet man, daß eigentlich nur das im Laserstrahl befindliche Volumen von ca. 2 x 2 x 10 mm (40 µl) zum Signal beiträgt, ergibt sich eine Nachweismenge von 0,4 nmol Fe.
Das ist in der Größenordnung der Nachweisempfindlichkeit des in DE 195 03 664 C2 beschriebenen Verfahrens. Der neue Meßplatz wurde nicht speziell auf hohe Nachweisempfindlichkeit optimiert. Die eingesetzten Teilchen enthalten nur eine sehr kleine Fraktion von Teilchen, die nach Brown ausgerichtet werden und damit zum Signal beitragen.

### 2. Messung des Einflusses der Zugabe von Biotin-BSA zu mit Streptavidin gekoppeltem Dextran-Magnetit auf die Relaxation der Doppelbrechung:

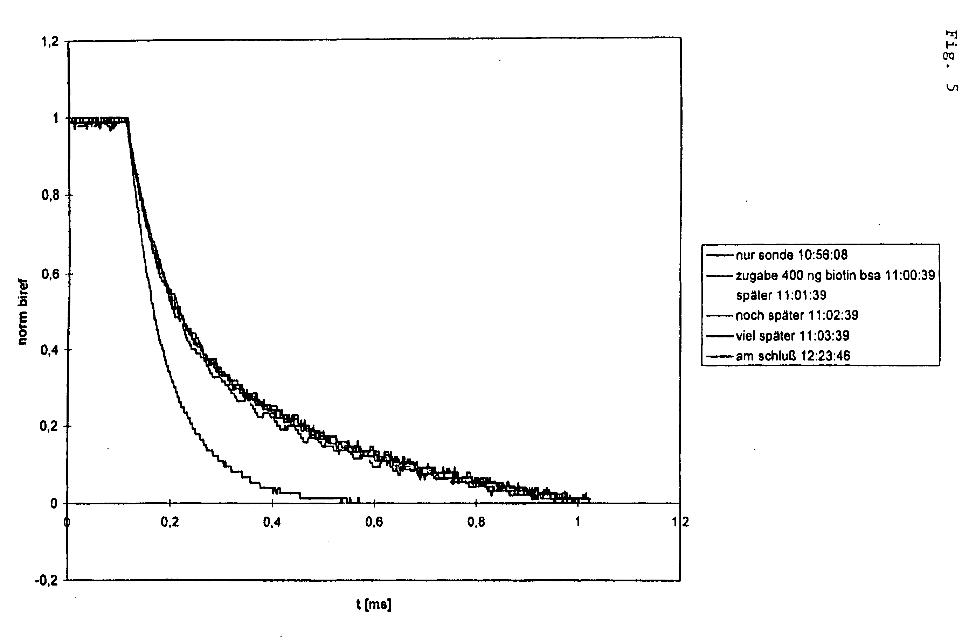

*Vorgehen:* Die Sonde (Dextran-Magnetit aus Beispiel 1, gekoppelt mit Streptavidin) lag in einer Ausgangskonzentration von 2,66 mmol Fe/l vor. Für die Messungen wurde eine Probe 1 bestehend aus 100 µl der Sonde verdünnt mit 400 µl BSA-PBS (PBS: Phosphate Buffered Saline) Puffer hergestellt. Das Relaxationssignal dieser Probe wurde gemessen.
Danach wurden einmalig 40 µl in BSA-PBS Puffer verdünntes Biotin-BSA (Absolutmenge 400 ng Biotin-BSA) zu dieser Probe zugegeben. Danach wurde zu verschiedenen Zeitpunkten nach Mischung das Relaxationssignal gemessen.
Als Kontrolle wurde eine Probe 2 bestehend aus 100 µl Dextran-Magnetit, gekoppelt mit Streptavidin (verdünnt in 400 µl BSA-PBS Puffer) mit 5 µl freiem Biotin (1:10 verdünnt, Ausgangskonzentration 1 mg/ml) abgesättigt. Auch hier wurde einmalig 40 µl in BSA-PBS Puffer verdünntes Biotin-BSA (Absolutmenge 400 ng Biotin-BSA) zugegeben, und zu verschiedenen Zeitpunkten nach Mischung das Relaxationssignal gemessen.
*Ergebnis:* Das Relaxationssignal von Probe 1 war deutlich zu beobachten. Nach Zugabe des Biotin-BSA zeigte sich eine signifikante Zunahme der Relaxationszeit (vgl. Fig. 5).
Diese Verlängerung der Relaxationszeit ist durch die Zunahme des hydrodynamischen Teilchendurchmessers der Sonde durch Biotin-BSA induzierte Aggregation bzw. Quervemetzung zu erklären.

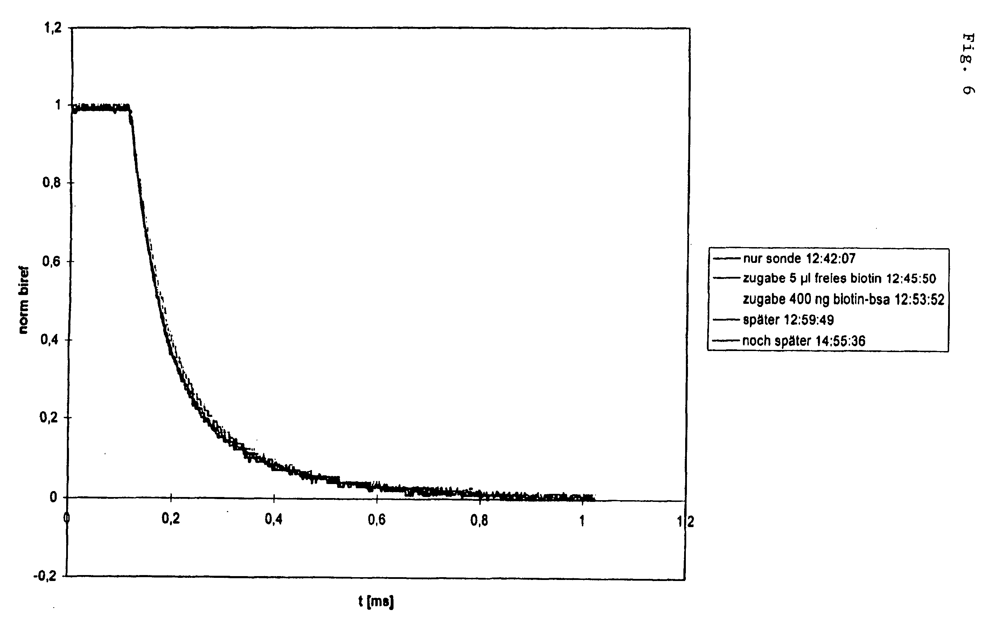

Auch Probe 2 zeigte ein deutliches Relaxationssignal. Die Zugabe des Biotin-BSA in BSA-PBS Puffer zu dieser Probe führte jedoch nicht zu einer Veränderung der Relaxationszeit (vgl. Fig.6).

Der daraus zu schließende unveränderte hydrodynamische Teilchendurchmesser zeigt, daß es durch die Absättigung der Bindungsstellen des Streptavidin mit freiem Biotin nicht mehr zu einer durch Biotin-BSA induzierten Aggregation bzw. Quervemetzung der Sonde kommt. Damit wurde die Spezifität der Bindungsreaktion bei Probe 1 nachgewiesen.

## Patentansprüche

1. Verfahren zur Detektion von Analyten bzw. Bindungsreaktionen, bei denen ferro- oder ferrimagnetische Substanzen als Markierung in Immunoassays oder sonstigen Bindungsassays eingesetzt werden, **dadurch gekennzeichnet, daß** die Relaxation der Doppelbrechung als Meßgröße bestimmt wird.

2. Verfahren zur Detektion von Analyten bzw. Bindungsreaktionen, bei denen ferro- oder ferrimagnetische Substanzen als Markierung in Immunoassays oder sonstigen Bindungsassays eingesetzt werden, **dadurch gekennzeichnet, daß** die Doppelbrechung im Frequenzbereich als Meßgröße bestimmt wird.

3. Verfahren zur Detektion von Analyten bzw. Bindungsreaktionen gemäß Anspruch 1 oder 2, **dadurch gekennzeichnet, daß** ferro- oder ferrimagnetische Substanzen als Markierung in Immunoassays oder sonstigen Bindungsassays eingesetzt werden, wobei die Brownsche Relaxation zumindest eines Teils dieser Substanzen unter den Meßbedingungen schneller verläuft als die Néelsche Relaxation.

4. Verfahren zur Detektion von Analyten bzw. Bindungsreaktionen, **dadurch gekennzeichnet, daß**
(i) die die Analyten bindenden strukturspezifischen Substanzen zunächst mit ferrimagnetischen oder ferromagnetischen Substanzen markiert werden und anschließend
(ii) diese markierten strukturspezifischen Substanzen in einer zu vermessenden Probe eingesetzt werden,
(iii) die zu vermessende Probe mittels eines von außen angelegten Magnetfeldes aufmagnetisiert wird und
(iv) nach Abschalten des äußeren Feldes die Relaxation der Doppelbrechung der magnetischen Marker vermessen wird.

5. Verfahren zur Detektion von Analyten bzw. Bindungsreaktionen, **dadurch gekennzeichnet, daß**
(i) Analyte zunächst mit ferrimagnetischen oder ferromagnetischen Substanzen markiert werden und anschließend
(ii) diese magnetisch markierten Analyte in einer zu vermessenden Probe eingesetzt werden, welcher Substanzen zugesetzt wurden, die die Analyte spezifisch binden, und
(iii) die zu vermessende Probe mittels eines von außen angelegten Magnetfeldes aufmagnetisiert wird und
(iv) nach Abschalten des äußeren Feldes die Relaxation der Doppelbrechung der magnetischen Marker vermessen wird.

6. Verfahren zur Detektion von in flüssiger Phase vorliegenden Analyten, **dadurch gekennzeichnet, daß**
(i) die die Analyten bindenden strukturspezifischen Substanzen zunächst mit ferrimagnetischen oder ferromagnetischen Substanzen markiert werden, wobei die Brownsche Relaxation zumindest eines Teils dieser Substanzen unter den Meßbedingungen eine kürzere Relaxationszeit aufweist als die Néelsche Relaxation und anschließend
(ii) diese magnetisch markierten Substanzen in einer zu vermessenden Probe eingesetzt werden, und
(iii) die zu vermessende Probe mittels eines von außen angelegten Magnetfeldes aufmagnetisiert wird und
(iv) nach Abschalten des äußeren Feldes die Relaxation der Doppelbrechung der magnetischen Marker vermessen wird, wobei das unterschiedliche Relaxationsverhalten der mit dem Analyten gebundenen gegenüber den ungebundenen magnetischen Markern zur Analyse herangezogen wird.

7. Verfahren zur Detektion von in flüssiger Phase vorliegenden Analyten, **dadurch gekennzeichnet, daß**
(i) Analyte zunächst mit ferrimagnetischen oder ferromagnetischen Substanzen markiert werden, wobei die Brownsche Relaxation zumindest eines Teils dieser Substanzen unter den Meßbedingungen eine kürzere Relaxationszeit aufweist als die Néelsche Relaxation und anschließend
(ii) diese magnetisch markierten Analyte in einer zu vermessenden Probe eingesetzt werden, welcher Substanzen zugesetzt wurden, die die Analyte spezifisch binden, und
(iii) die zu vermessende Probe mittels eines von außen angelegten Magnetfeldes aufmagnetisiert wird und
(iv) nach Abschalten des äußeren Feldes die Relaxation der Doppelbrechung der magnetischen Marker vermessen wird, wobei das unterschiedliche Relaxationsverhalten der mit dem Analyten gebundenen gegenüber den ungebundenen magnetischen Markern zur Analyse herangezogen wird.

8. Verfahren nach Anspruch 6, **dadurch gekennzeichnet, daß** den zu vermessenden Proben zusätzlich die Analyte spezifisch bindende Substanzen zugesetzt wurden.

9. Verfahren gemäß den Ansprüchen 4 bis 8, **dadurch gekennzeichnet, daß** zur Detektion anstelle der Messung der Relaxation der Doppelbrechung die Messung der Doppelbrechung im Frequenzbereich herangezogen wird.

10. Verfahren gemäß den Ansprüchen 1 bis 9, **dadurch gekennzeichnet, daß** die strukturspezifischen Substanzen Antikörper, Antikörperfragmente, Biotin oder Biotin spezifisch bindende Substanzen wie Avidin bzw. Streptavidin, Neutravidin oder Extravidin, spezifisch an Rezeptoren bindende Agonisten oder deren Antagonisten, spezifische Peptide und Proteine, Rezeptoren, Enzyme, Enzymsubstrate, Nukleotide, Ribonukleinsäuren, Desoxyribonukleinsäuren, Kohlenhydrate oder Lipoproteine sind.

11. Verfahren gemäß Anspruch 10, **dadurch gekennzeichnet, daß** die an Rezeptoren bindenden Agonisten Zytokine, Lymphokine oder Endotheline sind.

12. Verfahren gemäß den Ansprüchen 10 und 11, **dadurch gekennzeichnet, daß** die strukturspezifischen Substanzen eine Bindungskonstante im Bereich von 10⁵ - 10¹⁵ (mol/l)⁻¹ haben.

13. Verfahren gemäß den Ansprüchen 1 bis 12, **dadurch gekennzeichnet, daß** eine Bestimmung von zwei oder mehreren verschiedenen Analyten in einer Probe durchgeführt wird.

14. Verfahren gemäß Anspruch 13, **dadurch gekennzeichnet, daß** zwei oder mehrere ferromagnetische oder ferrimagnetische Substanzen mit diskriminierbaren Brownschen Relaxationszeiten verwendet werden.

15. Verfahren gemäß den Ansprüchen 1 bis 14, **dadurch gekennzeichnet, daß** die ferromagnetischen oder ferrimagnetischen Substanzen eine Teilchengröße im Bereich von Inm bis 100 µm haben.

16. Verfahren gemäß den Ansprüchen 1 bis 14, **dadurch gekennzeichnet, daß** die ferromagnetischen oder ferrimagnetischen Substanzen mit einer Hülle aus oligomeren oder polymeren Kohlenhydraten, Proteinen, Peptiden, Nukleotiden und/oder Lipiden stabilisiert sind.

17. Verwendung von Verbindungen aus Kombinationen von ferro- oder ferrimagnetischen Substanzen mit strukturspezifischen Substanzen zur Detektion von Analyten bzw. Bindungreaktionen mittels Messung der Relaxation der Doppelbrechung oder mittels Messung der Doppelbrechung im Frequenzbereich.

18. Verwendung von Verbindungen aus Kombinationen von ferro- oder ferrimagnetischen Substanzen mit nachzuweisenden Substanzen zur Detektion von Bindungreaktionen mittels Messung der Relaxation der Doppelbrechung oder mittels Messung der Doppelbrechung im Frequenzbereich.

19. Verwendung von Verbindungen, die aus Kombinationen von ferro- oder ferrimagnetischen Substanzen mit strukturspezifischen Substanzen bestehen, deren Brownsche Relaxation unter den Meßbedingungen schneller verläuft als die Néelsche Relaxation, zur Detektion von Analyten bzw. Bindungreaktionen mittels Messung der Relaxation der Doppelbrechung oder mittels Messung der Doppelbrechung im Frequenzbereich.

20. Verwendung von Verbindungen, die aus Kombinationen von ferro- oder ferrimagnetischen Substanzen mit nachzuweisenden Substanzen bestehen, deren Brownsche Relaxation unter den Meßbedingungen schneller verläuft als die Néelsche Relaxation, zur Detektion von Bindungreaktionen mittels Messung der Relaxation der Doppelbrechung oder mittels Messung der Doppelbrechung im Frequenzbereich.

21. Verwendung von Verbindungen in Verfahren gemäß den Ansprüchen 1 bis 14, **dadurch gekennzeichnet, daß** die Verbindungen ferromagnetische oder ferrimagnetische Substanzen enthalten, deren Teilchengröße im Bereich von 1 bis 100 µm liegt.

22. Verwendung von Verbindungen in Verfahren gemäß den Ansprüchen 1 bis 14, **dadurch gekennzeichnet, daß** die Verbindungen ferromagnetische oder ferrimagnetische Substanzen enthalten, die mit einer Hülle aus oligomeren oder polymeren Kohlenhydraten, Proteinen, Peptiden, Nukleotiden, Tensiden, Polymeren und/oder Lipiden stabilisiert sind.

23. Verwendung von Verbindungen in Verfahren gemäß den Ansprüchen 1 bis 14, **dadurch gekennzeichnet, daß** die Verbindungen strukturspezifische Substanzen enthalten, die Antikörper, Antikörperfragmente, Biotin oder Biotin bindende Substanzen wie Avidin oder Streptavidin, spezifisch an Rezeptoren bindende Agonisten oder deren Antagonisten, spezifische Peptide und Proteine, Rezeptoren, Enzyme, Enzymsubstrate, Nukleotide, Ribonukleinsäuren, Desoxyribonukleinsäuren, Kohlenhydrate oder Lipoproteine sind.

24. Verwendung der Verfahren gemäß den Ansprüchen 1 - 16 in der Fertilität, Histokompatibilität, Allergologie, Infektiologie, Hygiene, Genetik, Virologie, Bakteriologie, Toxikologie, Pathologie, Umweltanalytik, Lebensmittelchemie und medizinischen Diagnostik.

25. Verwendung von Kombinationen aus ferri- oder ferromagnetischen Substanzen mit strukturspezifischen Substanzen oder von Kombinationen aus ferri- oder ferromagnetischen Substanzen mit nachzuweisenden Analyten in Verfahren gemäß den Ansprüchen 1 - 16.

26. Verwendung einer Vorrichtung zur Durchführung der Verfahren gemäß einem der Ansprüche 1 bis 16, **dadurch gekennzeichnet, daß** die Vorrichtung eine Vorrichtung zur Erzeugung polarisierten Lichtes, eine Vorrichtung zur Aufnahme der Probe, eine Vorrichtung zur Aufmagnetisierung der Probe mit Magnetpulsen oder einem Magnetfeld variabler Frequenz, sowie eine Vorrichtung zur Analyse der Polarisationsrichtung polarisierten Lichtes enthält.

27. Verwendung einer Vorrichtung gemäß Anspruch 26, **dadurch gekennzeichnet, daß** auf einer optischen Bank ein Laser, ein Polarisator, eine Küvette mit der Probe, ein Analysator und ein Photodetektor angeordnet sind.

28. Verwendung einer Vorrichtung gemäß Anspruch 26 oder 27, **dadurch gekennzeichnet, daß** sich zusätzlich zwischen der Probe und dem Analysator ein λ/4-Plättchen befindet.

## Claims

1. Method of detection of analytes or binding reactions, in which ferromagnetic or ferrimagnetic substances are used as labelling in immunoassays or other binding assays, **characterized in that** birefringence relaxation is determined as the measured quantity.

2. Method of detection of analytes or binding reactions, in which ferromagnetic or ferrimagnetic substances are used as labelling in immunoassays or other binding assays, **characterized in that** the birefringence in the frequency domain is determined as the measured quantity.

3. Method of detection of analytes or binding reactions according to claim 1 or 2, **characterized in that** ferromagnetic or ferrimagnetic substances are used as labelling in immunoassays or other binding assays, and the Brownian relaxation of at least a portion of these substances proceeds faster than the Néel relaxation in the measurement conditions.

4. Method of detection of analytes or binding reactions, **characterized in that**
(i) the structure-specific substances binding the analytes are first labelled with ferrimagnetic or ferromagnetic substances and then
(ii) these labelled structur e-specific substances are used in a sample that is to be measured,
(iii) the sample to be measured is magnetized by means of an externally applied magnetic field and
(iv) after switching off the external field, the birefringence relaxation of the magnetic markers is measured.

5. Method of detection of analytes or binding reactions, **characterized in that**
(i) the structure-specific substances binding the analytes are first labelled with ferrimagnetic or ferromagnetic substances and then
(ii) these magnetically labelled analytes are used in a sample that is to be measured, to which substances are added that bind the analytes specifically, and
(iii) the sample to be measured is magnetized by means of an externally applied magnetic field and
(iv) after switching off the external field, the birefringence relaxation of the magnetic markers is measured.

6. Method of detection of analytes in the liquid phase, **characterized in that**
(i) the structure-specific substances binding the analytes are first labelled with ferrimagnetic or ferromagnetic substances, where the Brownian relaxation of at least a portion of these substances, in the measurement conditions, has a shorter relaxation time than the Néel relaxation and then
(ii) these magnetically labelled substances are used in a sample that is to be measured, and
(iii) the sample to be measured is magnetized by means of an externally applied magnetic field and
(iv) after switching off the external field, the birefringence relaxation of the magnetic markers is measured, with the different relaxation behaviour of the magnetic markers bound to the analytes relative to the unbound magnetic markers being employed for the analysis.

7. Method of detection of analytes in the liquid phase, **characterized in that**
(i) analytes are first labelled with ferrimagnetic or ferromagnetic substances, where the Brownian relaxation of at least a portion of these substances, in the measurement conditions, has a shorter relaxation time than the Néel relaxation and then
(ii) these magnetically labelled analytes are used in a sample that is to be measured, to which substances are added that bind the analytes specifically, and
(iii) the sample to be measured is magnetized by means of an externally applied magnetic field and
(iv) after switching off the external field, the birefringence relaxation of the magnetic markers is measured, with the different relaxation behaviour of the magnetic markers bound to the analytes relative to the unbound magnetic markers being employed for the analysis.

8. Method according to claim 6, **characterized in that** substances binding the analytes specifically are additionally added to the samples to be measured.

9. Method according to claims 4 to 8, **characterized in that** measurement of birefringence in the frequency domain is employed for detection instead of measurement of the birefringence relaxation.

10. Method according to claims 1 to 9, **characterized in that** the structure-specific substances are antibodies, antibody fragments, biotin or substances that bind biotin specifically such as avidin or streptavidin, neutravidin or extravidin, agonists binding specifically to receptors or their antagonists, specific peptides and proteins, receptors, enzymes, enzyme substrates, nucleotides, ribonucleic acids, deoxyribonucleic acids, carbohydrates or lipoproteins.

11. Method according to claim 10, **characterized in that** the agonists binding to receptors are cytokines, lymphokines or endothelins.

12. Method according to claims 10 and 11, **characterized in that** the structure-specific substances have a binding constant in the range 10⁵ - 10¹⁵ (mol/l)⁻¹.

13. Method according to claims 1 to 12, **characterized in that** determination of two or more different analytes in one sample is carried out.

14. Method according to claim 13, **characterized in that** two or more ferromagnetic or ferrimagnetic substances with distinguishable Brownian relaxation times are used.

15. Method according to claims 1 to 14, **characterized in that** the ferromagnetic or ferrimagnetic substances have a particle size in the range from 1 nm to 100 µm.

16. Method according to claims 1 to 14, **characterized in that** the ferromagnetic or ferrimagnetic substances are stabilized with an envelope of oligomeric or polymeric carbohydrates, proteins, peptides, nucleotides and/or lipids.

17. Use of combinations of ferromagnetic or ferrimagnetic substances with structure-specific substances for detection of analytes or binding reactions by measuring the birefringence relaxation or by measuring the birefringence in the frequency domain.

18. Use of combinations of ferromagnetic or ferrimagnetic substances with substances that are to be detected, for the detection of binding reactions by measuring the birefringence relaxation or by measuring the birefringence in the frequency domain.

19. Use of compounds that consist of combinations of ferromagnetic or ferrimagnetic substances with structure-specific substances, whose Brownian relaxation, in the measurement conditions, proceeds faster than the Néel relaxation, for the detection of analytes or binding reactions by measuring the birefringence relaxation or by measuring the birefringence in the frequency domain.

20. Use of compounds that consist of combinations of ferromagnetic or ferrimagnetic substances with substances that are to be detected, whose Brownian relaxation, in the measurement conditions, proceeds faster than the Néel relaxation, for the detection of binding reactions by measuring the birefringence relaxation or by measuring the birefringence in the frequency domain.

21. Use of compounds in methods according to claims 1 to 14, **characterized in that** the compounds contain ferromagnetic or ferrimagnetic substances whose particle size is in the range from 1 to 100 µm.

22. Use of compounds in methods according to claims 1 to 14, **characterized in that** the compounds contain ferromagnetic or ferrimagnetic substances that are stabilized with an envelope of oligomeric or polymeric carbohydrates, proteins, peptides, nucleotides, surfactants, polymers and/or lipids.

23. Use of compounds in methods according to claims 1 to 14, **characterized in that** the compounds contain structure-specific substances which are antibodies, antibody fragments, biotin or biotin-binding substances such as avidin or streptavidin, agonists that bind specifically to receptors, or their antagonists, specific peptides and proteins, receptors, enzymes, enzyme substrates, nucleotides, ribonucleic acids, deoxyribonucleic acids, carbohydrates or lipoproteins.

24. Use of methods according to claims 1-16 in fertility, histocompatibility, allergology, infectiology, hygiene, genetics, virology, bacteriology, toxicology, pathology, environmental analysis, food chemistry and medical diagnostics.

25. Use of combinations of ferrimagnetic or ferromagnetic substances with structure-specific substances or of combinations of ferrimagnetic or ferromagnetic substances with analytes that are to be detected, in methods according to claims 1-16.

26. Use of a device for carrying out the methods according to one of the claims 1 to 16, **characterized in that** the device comprises a device for producing polarized light, a device for holding the sample, a device for magnetizing the sample with magnetic pulses or a magnetic field of variable frequency, and a device for analysing the direction of polarization of polarized light.

27. Use of a device according to claim 26, **characterized in that** a laser, a polarizer, a cell containing the sample, an analyser and a photodetector are arranged on an optical bench.

28. Use of a device acc ording to claim 26 or 27, **characterized in that** there is additionally a λ/4 plate between the sample and the analyser.

## Revendications

1. Procédé en vue de la détection d'analytes ou de réactions de liaison, dans le cas desquels l'on utilise des substances ferromagnétiques ou ferrimagnétiques en tant que marquage dans des dosages immunologiques ou dans des dosages de liaison quelconques, **caractérisé en ce que** la relaxation de la biréfringence est déterminée en tant que grandeur de mesure.

2. Procédé en vue de la détection d'analytes ou de réactions de liaison, dans le cas desquels l'on utilise des substances ferromagnétiques ou ferrimagnétiques en tant que marquage dans des dosages immunologiques ou dans des dosages de liaison quelconques, **caractérisé en ce que** la biréfringence dans le domaine des fréquences est déterminée en tant que grandeur de mesure.

3. Procédé en vue de la détection d'analytes ou de réactions de liaison selon la revendication 1 ou 2, **caractérisé en ce que** l'on utilise des substances ferromagnétiques ou ferrimagnétiques en tant que marquage dans des dosages immunologiques ou des dosages de liaison quelconques, la relaxation de Brown d'au moins une partie de ces substances se déroulant plus rapidement dans les conditions de mesure que la relaxation de Néel.

4. Procédé en vue de la détection d'analytes ou de réactions de liaison, **caractérisé**
(i) en ce que les substances à structure spécifique liant les analytes sont marquées tout d'abord à l'aide de substances ferrimagnétiques ou ferromagnétiques et ensuite
(ii) en ce que ces substances marquées à structure spécifique sont utilisées dans un échantillon à mesurer,
(iii)en ce que l'échantillon à mesurer est magnétisé par l'application de l'extérieur d'un champ magnétique et
(iv) en ce qu'après l'interruption du champ externe, on mesure la relaxation de la biréfringence des marqueurs magnétiques.

5. Procédé en vue de la détection d'analytes ou de réactions de liaison, **caractérisé**
(i) en ce que les analytes sont marqués tout d'abord à l'aide de substances ferrimagnétiques ou ferromagnétiques et ensuite
(ii) en ce que l'on utilise ces analytes marqués de manière magnétique dans un échantillon à mesurer, auquel des substances ont été ajoutées, qui se lient de manière spécifique aux analytes, et
(iii)en ce que l'échantillon à mesurer est magnétisé par l'application de l'extérieur d'un champ magnétique et
(iv) en ce qu'après l'interruption du champ externe, on mesure la relaxation de la biréfringence des marqueurs magnétiques.

6. Procédé en vue de la détection d'analytes présents en phase liquide, **caractérisé**
(i) en ce que les substances à structure spécifique liant les analytes sont marquées tout d'abord à l'aide de substances ferrimagnétiques ou ferromagnétiques, la relaxation de Brown d'au moins une partie de ces substances dans les conditions de mesure présentant un temps de relaxation plus court que la relaxation de Néel et ensuite
(ii) en ce que ces substances marquées de manière magnétique sont utilisées dans un échantillon à mesurer,
(iii)en ce que l'échantillon à mesurer est magnétisé par l'application de l'extérieur d'un champ magnétique et
(iv) en ce qu'après l'interruption du champ externe, la relaxation de la biréfringence des marqueurs magnétiques est mesurée, le comportement de relaxation différent des marqueurs magnétiques non liés par rapport aux marqueurs liés aux analytes étant mis à profit en vue de l'analyse.

7. Procédé en vue de la détection d' analytes présents en phase liquide, **caractérisé**
(i) en ce que les analytes sont marquées tout d'abord à l'aide de substances ferrimagnétiques ou ferromagnétiques, la relaxation de Brown d'au moins une partie de ces substances présentant, dans les conditions de mesure, un temps de relaxation plus court que la relaxation de Néel et ensuite
(ii) en ce que l'on utilise ces analytes marqués de manière magnétique dans un échantillon à mesurer, auquel des substances ont été ajoutées, qui se lient de manière spécifique aux analytes, et
(iii)en ce que l'échantillon à mesurer est magnétisé par l'application de l'extérieur d'un champ magnétique et
(iv) en ce qu'après l'interruption du champ externe, la relaxation de la biréfringence des marqueurs magnétiques est mesurée, le comportement de relaxation différent des marqueurs magnétiques non liés par rapport aux marqueurs liés aux analytes étant mis à profit en vue de l'analyse.

8. Procédé selon la revendication 6, **caractérisé en ce que** l'on ajoute aux échantillons à mesurer en outre des substances liant spécifiquement les analytes.

9. Procédé selon les revendications 4 à 8, **caractérisé en ce qu'**en vue de la détection, on met à profit, au lieu de la mesure de la relaxation de la biréfringence, la mesure de la biréfringence dans le domaine des fréquences.

10. Procédé selon les revendications 1 à 9, **caractérisé en ce que** les substances à structure spécifique sont des anticorps, des fragments d'anticorps, la biotine ou des substances liant spécifiquement la biotine, comme l'avidine ou la streptavidine, la neutravidine ou l'extravidine, des agonistes se liant de manière spécifique à des récepteurs ou leurs antagonistes, des peptides et des protéines spécifiques, des récepteurs, des enzymes, des substrats enzymatiques, des nucléotides, des acides ribonucléiques, des acides désoxyribonucléiques, des hydrates de carbone ou des lipoprotéines.

11. Procédé selon la revendication 10, **caractérisé en ce que** les agonistes se liant à des récepteurs sont des cytokines, des lymphokines ou des endothélines.

12. Procédé selon les revendications 10 et 11, **caractérisé en ce que** les substances à structure spécifique ont une constante de liaison dans le domaine de 10⁵ - 10¹⁵ (moles/l)⁻¹.

13. Procédé selon les revendications 1 à 12, **caractérisé en ce que** l'on effectue, dans un échantillon, une détermination de deux ou de plusieurs analytes divers.

14. Procédé selon la revendication 13, **caractérisé en ce que** l'on utilise deux ou plusieurs substances ferromagnétiques ou ferrimagnétiques ayant des temps de relaxation browniens capables d'être discriminés.

15. Procédé selon les revendications 1 à 14, **caractérisé en ce que** les substances ferromagnétiques ou ferrimagnétiques possèdent une grosseur de particule dans le domaine de 1 nm à 100 µm.

16. Procédé selon les revendications 1 à 14, **caractérisé en ce que** les substances ferromagnétiques ou ferrimagnétiques sont stabilisées à l'aide d'une enveloppe faite à partir d'hydrates de carbone oligomères ou polymères, de protéines, de peptides, de nucléotides, et/ou de lipides.

17. Utilisation de composés faits à partir de combinaisons de substances ferromagnétiques ou ferrimagnétiques avec des substances à structure spécifique, en vue de la détection d'analytes ou de réactions de liaison par l'intermédiaire de la mesure de la relaxation de la biréfringence ou par l'intermédiaire de la mesure de la biréfringence dans le domaine des fréquences.

18. Utilisation de composés faits à partir de combinaisons de substances ferromagnétiques ou ferrimagnétiques avec des substances à détecter, en vue de la détection de réactions de liaison par l'intermédiaire de la mesure de la relaxation de la biréfringence ou par l'intermédiaire de la mesure de la biréfringence dans le domaine des fréquences.

19. Utilisation de composés, qui se composent de combinaisons de substances ferromagnétiques ou ferrimagnétiques avec des substances à structure spécifique, dont la relaxation de Brown se déroule plus rapidement dans les conditions de mesure que la relaxation de Néel, en vue de la détection d'analytes ou de réactions de liaison par l'intermédiaire de la mesure de la relaxation de la biréfringence ou par l'intermédiaire de la mesure de la biréfringence dans le domaine des fréquences.

20. Utilisation de composés, qui se composent de combinaisons de substances ferromagnétiques ou ferrimagnétiques avec des substances à détecter, dont la relaxation de Brown se déroule plus rapidement dans les conditions de mesure que la relaxation de Néel, en vue de la détection de réactions de liaison par l'intermédiaire de la mesure de la relaxation de la biréfringence ou par l'intermédiaire de la mesure de la biréfringence dans le domaine des fréquences.

21. Utilisation de composés dans des procédés selon les revendications 1 à 14, **caractérisée en ce que** les composés contiennent des substances ferromagnétiques ou ferrimagnétiques, dont la grosseur de particule se situe dans le domaine de 1 à 100 µm.

22. Utilisation de composés dans des procédés selon les revendications 1 à 14, **caractérisée en ce que** les composés contiennent des substances ferromagnétiques ou ferrimagnétiques, qui sont stabilisées à l'aide d'une enveloppe faite à partir d'hydrates de carbone oligomères ou polymères, de protéines, de peptides, de nucléotides, d'agents tensioactifs, de polymères et/ou de lipides.

23. Utilisation de composés dans des procédés selon les revendications 1 à 14, **caractérisée en ce que** les composés contiennent des substances à structure spécifique, qui sont des anticorps, des fragments d'anticorps, la biotine ou des substances liant la biotine, comme l'avidine ou la streptavidine, des agonistes se liant de manière spécifique à des récepteurs ou leurs antagonistes, des peptides et des protéines spécifiques, des récepteurs, des enzymes, des substrats enzymatiques, des nucléotides, des acides ribonucléiques, des acides désoxyribonucléiques, des hydrates de carbone ou des lipoprotéines.

24. Utilisation de procédés selon les revendications 1 à 16 dans les domaines de la fertilité, de l'histocompatibilité, de l'allergologie, de l'infectiologie, de l'hygiène, de la génétique, de la virologie, de la bactériologie, de la toxicologie, de la pathologie, de l'analytique de l'environnement, de la chimie des produits alimentaires et de la diagnostique médicale.

25. Utilisation de combinaisons faites de substances ferromagnétiques ou ferrimagnétiques avec des substances à structure spécifique ou de combinaisons faites de substances ferromagnétiques ou ferrimagnétiques avec des analytes à détecter dans des procédés selon les revendications 1 à 16.

26. Utilisation d'un dispositif en vue de l'exécution de procédés selon l'une des revendications 1 à 16, **caractérisée en ce que** le dispositif contient un dispositif en vue de la production de lumière polarisée, un dispositif en vue de la réception d'un échantillon, un dispositif en vue de la magnétisation de l'échantillon à l'aide de pulsations magnétiques ou d'un champ magnétique de fréquence variable ainsi qu'un dispositif en vue de l'analyse de la direction de polarisation de la lumière polarisée.

27. Utilisation d'un dispositif selon la revendication 26, **caractérisée en ce que** l'on dispose, sur un banc d'optique, un laser, un polarisateur, une cuvette avec l'échantillon, un analyseur et un photo détecteur.

28. Utilisation d'un dispositif selon la revendication 26 ou 27, **caractérisée en ce que** se trouve en plus entre l'échantillon et l'analyseur une plaquette λ/4.
